(19) Europäisches Patentamt / European Patent Office / Office européen des brevets

(11) **EP 4 759 295 A1**

(12) **EUROPEAN PATENT APPLICATION**
published in accordance with Art. 153(4) EPC


(43) Date of publication:
**17.06.2026 Bulletin 2026/25**

(21) Application number: **24851916.7**

(22) Date of filing: **08.08.2024**

(51) International Patent Classification (IPC):
***A61K 8/19*** *(2006.01)* ***A61K 8/06*** *(2006.01)*
***A61K 8/37*** *(2006.01)* ***A61K 8/891*** *(2006.01)*
***A61K 8/892*** *(2006.01)* ***A61K 8/893*** *(2006.01)*
***A61Q 1/02*** *(2006.01)*

(52) Cooperative Patent Classification (CPC):
**A61K 8/02; A61K 8/06; A61K 8/19; A61K 8/25; A61K 8/37; A61K 8/891; A61K 8/892; A61K 8/893; A61K 8/894; A61Q 1/02**

(86) International application number:
**PCT/JP2024/028456**

(87) International publication number:
**WO 2025/033499 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.08.2023 JP 2023131142**



(71) Applicant: **Kao Corporation**
**Tokyo 103-8210 (JP)**

(72) Inventor: **TATEBE, Momoko**
**Tokyo 103-8210 (JP)**

(74) Representative: **Hoffmann Eitle**
**Patent- und Rechtsanwälte PartmbB**
**Arabellastraße 30**
**81925 München (DE)**


(54) **WATER-IN-OIL EMULSION COMPOSITION**


(57) Provided is a water-in-oil emulsion composition for impregnation comprising the following components (A), (B), (C) and (D): (A) cation-modified clay mineral substituted with a quaternary ammonium ion, (B) one or more members selected from the group consisting of phenyl-modified silicone and alkyl-modified silicone which are in a liquid state at 25°C, (C) modified silicone containing an alkyl group having 10 or more carbon atoms and a polyglyceryl group, and (D) an ester oil which is in a liquid state at 25°C.


EP 4 759 295 A1

## Description

Field of the Invention

The present invention relates to a water-in-oil emulsion composition.

Background of the Invention

Heretofore, cosmetics have been developed in a form in which a holder, such as sponge, housed in a compact container is impregnated with a liquid cosmetic, and the liquid cosmetic is taken from the holder by finger or an applicator when using to be applied to the skin upon application.
For example, Patent Literature 1 states that a cosmetic product containing a polyether urethane foam impregnated with a cosmetic composition is excellent in stability, portability, and use impression.
Patent Literature 2 states that a cosmetic product having water-insoluble sponge impregnated with a cosmetic composition containing a specific organic ultraviolet shielding agent is excellent in ultraviolet shielding function.
Patent Literature 3 states that a water-in-oil emulsion cosmetic for impregnation containing cross-linked polyether-modified silicone or cross-linked polyglycerin-modified silicone and an acrylic polymer having a dendrimeric siloxane structure in a side chain or silicone-modified pullulan is excellent in use impression and effect of refining skin pores and the like while maintaining appropriate filling to an impregnated material and holdability.
(Patent Literature 1) JP-A-2013-530252
(Patent Literature 2) JP-A-2017-088631
(Patent Literature 3) JP-A-2019-131489

Summary of the Invention

The present invention relates to a water-in-oil emulsion composition for impregnation comprising the following components (A), (B), (C) and (D):

(A) cation-modified clay mineral substituted by a quaternary ammonium ion,
(B) one or more members selected from the group consisting of phenyl-modified silicone and alkyl-modified silicone which are in a liquid state at 25°C,
(C) modified silicone containing an alkyl group having 10 or more carbon atoms and a polyglyceryl group, and
(D) an ester oil which is in a liquid state at 25°C.

The present invention also relates to a cosmetic in which a holder impregnated with the water-in-oil emulsion composition is housed in a compact container.
The present invention also relates to a method for using the cosmetic, characterized by taking a water-in-oil emulsion composition impregnated into a holder by finger or an applicator from the holder, and applying it to the skin.
The present invention also relates to a cosmetic kit comprising a set of a holder impregnated with a water-in-oil emulsion composition and a compact container, the water-in-oil emulsion composition comprising the following components (A), (B), (C) and (D):

(A) cation-modified clay mineral substituted with a quaternary ammonium ion,
(B) one or more members selected from the group consisting of phenyl-modified silicone and alkyl-modified silicone which are in a liquid state at 25°C,
(C) modified silicone containing an alkyl group having 10 or more carbon atoms and a polyglyceryl group, and
(D) an ester oil which is in a liquid state at 25°C.

The present invention also relates to a water-in-oil emulsion composition comprising the following components (A), (B), (C) and (D) :

(A) cation-modified clay mineral substituted with a quaternary ammonium ion,
(B) one or more members selected from the group consisting of phenyl-modified silicone and alkyl-modified silicone which are in a liquid state at 25°C,
(C) modified silicone containing an alkyl group having 10 or more carbon atoms and a polyglyceryl group, and
(D) an ester oil which is in a liquid state at 25°C.

Detailed Description of the Invention

Problems of conventional cosmetics are poor adhesiveness to the skin, likeliness to cause shine and makeup wear-off, and poor makeup durability.

The present inventor found that a water-in-oil emulsion composition comprising cation-modified clay mineral, one or more members selected from the group consisting of phenyl-modified silicone and alkyl-modified silicone, alkyl glyceryl ether-modified silicone, and a liquid ester oil in combination improves adhesiveness to the skin, offers favorable fit feeling to the skin, is unlikely to cause makeup wear-off, is excellent in makeup durability, and is also excellent in emulsification stability.

The water-in-oil emulsion composition of the present invention improves adhesiveness to the skin, offers favorable fit feeling to the skin, is free from makeup wear-off even after a lapse of a long time, is excellent in makeup durability, and is also excellent in emulsification stability. The water-in-oil emulsion composition is suitable for use as a cosmetic in which a holder impregnated with the water-in-oil emulsion composition is housed in a compact container.

The cation-modified clay mineral as the component (A) used in the present invention can be used without any limitation as long as it has substitution by a quaternary ammonium ion and can be used in usual cosmetics. For example, cation-modified clay mineral is preferred which is obtained by substituting layered clay mineral such as bentonite, laponite, hectorite, montmorillonite, or magnesium aluminum silicate by a quaternary ammonium salt cationic surfactant.

In this context, the quaternary ammonium salt cationic surfactant is of the following formula (1):

$$(R^1R^2R^3R^4N)^+X^- \qquad (1)$$

wherein $R^1$ represents an alkyl group having 10 to 22 carbon atoms or a benzyl group, $R^2$ represents a methyl group or an alkyl group having 10 to 22 carbon atoms, each of $R^3$ and $R^4$ represents an alkyl group or a hydroxyalkyl group having 1 to 3 carbon atoms, and X represents a halogen atom or a methyl sulfate residue.

Specific examples thereof include dodecyltrimethyl ammonium chloride, myristyltrimethyl ammonium chloride, cetyltrimethyl ammonium chloride, stearyltrimethyl ammonium chloride, behenyltrimethyl ammonium chloride, myristyldimethylethyl ammonium chloride, cetyldimethylethyl ammonium chloride, stearyldimethylethyl ammonium chloride, behenyldimethylethyl ammonium chloride, myristyldiethylmethyl ammonium chloride, cetyldiethylmethyl ammonium chloride, stearyldiethylmethyl ammonium chloride, behenyldiethylmethyl ammonium chloride, benzyldimethylmyristyl ammonium chloride, benzyldimethylcetyl ammonium chloride, benzyldimethylstearyl ammonium chloride, benzyldimethylbehenyl ammonium chloride, benzylmethylethylcetyl ammonium chloride, benzylmethylethylstearyl ammonium chloride, distearyldimethylammonium chloride, dibehenyldihydroxyethyl ammonium chloride, and compounds of each of these compounds with bromide instead of chloride, and further, dipalmitylpropylethyl ammonium methyl sulfate.

Among them, the quaternary ammonium salt cationic surfactant preferably comprises one or more members selected from the group consisting of benzyldimethylstearyl ammonium chloride, and dimethyldistearyl ammonium chloride, and is more preferably dimethyldistearyl ammonium chloride.

The cation-modified clay mineral obtained by substituting the layered clay mineral by the quaternary ammonium salt cationic surfactant preferably comprises one or more members selected from the group consisting of dimethyldistearyl ammonium hectorite, dimethyldistearyl ammonium bentonite, and benzyldimethylstearyl ammonium hectorite, more preferably comprises one or more members selected from the group consisting of dimethyldistearyl ammonium hectorite and benzyldimethylstearyl ammonium hectorite, and further more preferably comprises dimethyldistearyl ammonium hectorite. Examples of the commercially available product thereof include BENTONE 38, BENTONE 38VCG, and BENTONE 27 (all manufactured by Elementis Japan Co., Ltd.).

The organic modified clay mineral may be diluted with a solvent and used as a dispersion from the viewpoint of improvement in workability and an excellent thickening effect on an oil.

Specifically, a premix gel of the organic modified clay mineral dispersed in a solvent in advance is preferably used. The solvent is not limited as long as it is capable of being thickened by the organic modified clay mineral. The solvent preferably comprises one or more members selected from the group consisting of a hydrocarbon oil such as C9-12 alkane, C13-15 alkane, and a mineral oil, a silicone oil such as phenyl trimethicone, glyceryl tri(caprylate/caprate), and alkyl (C12-15) benzoate, and more preferably comprises one or more members selected from the group consisting of glyceryl tri(caprylate/caprate) and alkyl (C12-15) benzoate, from the viewpoint of a thickening effect on an oil.

The content of the organic modified clay mineral in the premix gel is preferably from 5 to 25% by mass, more preferably from 10 to 20% by mass, further more preferably from 10 to 18% by mass, from the viewpoint of improvement in workability and from the viewpoint of a thickening effect on an oil and the suppression of the oil separation of a thickened oily gel itself.

A commercially available product such as BENTONE GEL LC V, BENTONE GEL HS V, BENTONE LUXE XO, BENTONE GEL MIOV, BENTONE GEL VS-5 PC V, or BENTONE GEL PTM V (all manufactured by Elementis Specialties, Inc.), or BENTONE GEL GTCC V or BENTONE GEL TN V (all manufactured by Elementis Specialties, Inc.) can be used as

the premix gel.

One of or two or more in combination of these components (A) can be used. The content is preferably 0.1% by mass or more, more preferably 0.3% by mass or more, further more preferably 0.5% by mass or more, and preferably 8% by mass or less, more preferably 4% by mass or less, further more preferably 2% by mass or less, in terms of a solid content in the entire composition of the water-in-oil emulsion composition from the viewpoint of being excellent in adhesiveness to the skin, coverage after application, and stability. The content of the component (A) is preferably from 0.1 to 8% by mass, more preferably from 0.3 to 4% by mass, further more preferably from 0.5 to 2% by mass, in terms of a solid content in the entire composition of the water-in-oil emulsion composition.

The component (B) used in the present invention is one or more members selected from the group consisting of phenyl-modified silicone and alkyl-modified silicone which are in a liquid state at 25°C.

The liquid state at 25°C refers to having a viscosity of 10 000 mPa·s or less at 25°C.

The viscosity at 25°C of the component (B) is preferably 4 000 mPa·s or less, more preferably 600 mPa·s or less, further more preferably 200 mPa·s or less, even more preferably 50 mPa·s or less.

In this context, the viscosity is measured under the following conditions.

Viscosity measurement conditions: B type viscometer (TVB-10 model, manufactured by Toki Sangyo Co., Ltd.), rotor No. 4, 60 rpm, 60 sec.

Examples of the phenyl-modified silicone which is in a liquid state at 25°C as the component (B) include methylphenylpolysiloxane, phenyl trimethicone, diphenylsiloxyphenyl trimethicone, diphenyl dimethicone, trimethylsiloxyphenyl dimethicone, and trimethylpentaphenyltrisiloxane.

Among them, the phenyl-modified silicone preferably comprises one or more members selected from the group consisting of methylphenylpolysiloxane, phenyl trimethicone, and diphenylsiloxyphenyl trimethicone, and more preferably comprises methylphenylpolysiloxane, from the viewpoint of excellent adhesiveness of a coating film after application.

A commercially available product can be used as the phenyl-modified silicone which is in a liquid state at 25°C, for example: SH556 Fluid (manufactured by DuPont Toray Specialty Materials K.K.) as phenyl trimethicone; KF-56A (manufactured by Shin-Etsu Chemical Co., Ltd.) as diphenylsiloxyphenyl trimethicone; DOWSIL FZ-209 (manufactured by Dow Toray Co., Ltd.) as methylphenylpolysiloxane; KF-50 (manufactured by Shin-Etsu Chemical Co., Ltd.), KF-53 (manufactured by Shin-Etsu Chemical Co., Ltd.), or KF-54P (manufactured by Shin-Etsu Chemical Co., Ltd.) as diphenyl dimethicone; PDM-1000 (manufactured by Wacker Asahikasei Silicone Co., Ltd.) as trimethylsiloxyphenyl dimethicone; or PH-1555 HRI Cosmetic Fluid (manufactured by DuPont Toray Specialty Materials K.K.) as trimethylpentaphenyltrisiloxane.

Examples of the alkyl-modified silicone which is in a liquid state at 25°C as the component (B) include silicone oils with an added alkyl group having 8 to 28 carbon atoms. The alkyl group preferably has 8 to 24 carbon atoms, more preferably 8 to 20 carbon atoms, further more preferably 8 to 16 carbon atoms, even more preferably 8 to 12 carbon atoms, from the viewpoint of excellent conformability with the skin.

Examples of the alkyl-modified silicone which is in a liquid state at 25°C include caprylyl methicone, cetyl dimethicone, alkyl (C26-28) dimethicone, stearyl dimethicone, behenoxy dimethicone, and (acrylates/stearyl acrylate/dimethicone methacrylate) copolymers which are used in usual cosmetics.

Among them, the alkyl-modified silicone preferably comprises at least one or more members selected from the group consisting of caprylyl methicone, cetyl dimethicone, alkyl (C26-28) dimethicone, stearyl dimethicone, and behenoxy dimethicone, and more preferably comprises at least caprylyl methicone.

The phenyl-modified silicone and the alkyl-modified silicone which are in a liquid state at 25°C are preferably used in combination as the component (B) from the viewpoint of adhesiveness of a coating film after application and excellent conformability with the skin.

One or more of these components (B) can be used. The content is preferably 1% by mass or more, more preferably 6% by mass or more, further more preferably 11% by mass or more, and preferably 30% by mass or less, more preferably 21% by mass or less, further more preferably 16% by mass or less, in the entire composition of the water-in-oil emulsion composition from the viewpoint of adhesiveness of a coating film after application and excellent conformability with the skin. The content of the component (B) is preferably from 1 to 30% by mass, more preferably from 6 to 21% by mass, further more preferably from 11 to 16% by mass, in the entire composition of the water-in-oil emulsion composition.

The component (C) is modified silicone having an alkyl group having 10 or more carbon atoms and a polyglyceryl group, and has a siloxane skeleton, an alkyl group having 10 or more carbon atoms, and a polyoxyalkylene group or a polyglyceryl group.

The siloxane skeleton refers to a so-called silicone chain. Examples thereof include dimethylpolysiloxane (dimethicone), methylphenylpolysiloxane, and methyl hydrogen polysiloxane. The silicone chain may not be particularly limited by its length as long as the length can be usually used in cosmetics and the like.

The modified silicone as the component (C) may be a block copolymer comprising a polyoxyalkylene group or polyglyceryl group chain and a silicone chain in the backbone, may be terminally modified type, or may be side chain modified type in which a polyoxyalkylene group or a polyglyceryl group is bonded to a side chain of the siloxane skeleton.

Oxyalkylene of the polyoxyalkylene group contained in the modified silicone has one or more of ethylene oxide and propylene oxide in one molecule, and preferably has ethylene oxide and propylene oxide. The number of moles of oxyalkylene added in the polyoxyalkylene group is preferably from 4 to 12, more preferably from 8 to 10. The number of moles of glycerin added in the polyglyceryl group contained in the modified silicone is preferably from 1 to 10, more preferably from 1 to 4.
The modified silicone as the component (C) preferably has HLB of from 2 to 7, more preferably from 2 to 5, from the viewpoint of spread with a uniform thickness upon spreading, the prevention of makeup wear-off, shine ascribable to sebum, and the like, and improvement in makeup durability.
In the present invention, the HLB (hydrophilic-lipophilic balance) refers to the proportion of the molecular weight of a hydrophilic group moiety to the total molecular weight of a surfactant, and is determined in accordance with the following Griffin's equation.

HLB = 20 $\times$ (Molecular weight of a hydrophilic group / Total molecular weight)

Specific examples of the modified silicone as the component (C) include lauryl PEG-10 tris(trimethylsiloxy) silylethyl dimethicone, cetyl diglyceryl tris(trimethylsiloxy)silylethyl dimethicone, cetyl PEG/PPG-10/1 dimethicone, lauryl PEG-9 polydimethylsiloxyethyl dimethicone, and lauryl PEG/PPG-18/18 methicone.
Examples of the modified silicone as the component (C) include alkyl glyceryl ether-modified silicone of the following formula (2):

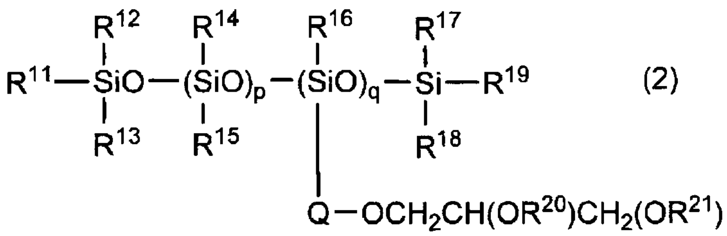
(2)

wherein $R^{11}$ and $R^{19}$ are the same or different and are each independently a linear or branched hydrocarbon group having 1 to 32 carbon atoms in terms of a mode value; $R^{12}$ to $R^{18}$ are the same or different and are each independently a linear or branched hydrocarbon group having 1 to 5 carbon atoms; Q is a linear or branched divalent hydrocarbon group having 3 to 20 carbon atoms; $R^{20}$ and $R^{21}$ are each independently a hydrogen atom or a linear or branched hydrocarbon group having 1 to 28 carbon atoms, and at least one of them is a hydrogen atom; p represents the number of repeat units and is a number of 5 or more and 60 or less as an average value; q represents the number of repeat units and is a number of 2.5 or more and 10 or less as an average value; and constituent units with the number of repeats, p and q, are any of block copolymers and random copolymers, provided that the formula has one or more alkyl groups having 10 or more carbon atoms.
In the formula (2), $R^{11}$ and $R^{19}$ are each independently a linear or branched hydrocarbon group having 1 to 32 carbon atoms in terms of a mode value. In this context, the mode value of the number of carbon atoms refers to the number of carbon atoms of the most abundant hydrocarbon group in chain lengths of a hydrocarbon group having a distribution The mode value of the number of carbon atoms is preferably from 8 to 32, more preferably from 12 to 28, further more preferably from 16 to 18.
In the formula (2), $R^{12}$ to $R^{18}$ each independently represent a linear or branched hydrocarbon group having 1 to 5 carbon atoms, and may be the same or different. Use of the hydrocarbon group having the number of carbon atoms within this range can effectively prevent a coating film from being plasticized due to an oil agent contained in food. Specific examples thereof include: linear alkyl groups such as a methyl group, an ethyl group, an n-propyl group, an n-butyl group, and a pentyl group; and branched alkyl groups such as an isopropyl group, a sec-butyl group, a tert-butyl group, and a neopentyl group. Among them, a methyl group is preferred from the viewpoint of easy availability.
In the formula (2), Q represents a linear or branched divalent hydrocarbon group having 3 to 20 carbon atoms.
Examples of the divalent hydrocarbon group having 3 to 20 carbon atoms represented by Q include: linear alkylene groups such as a trimethylene group, a tetramethylene group, a pentamethylene group, a hexamethylene group, a heptamethylene group, an octamethylene group, a nonamethylene group, a decamethylene group, an undecamethylene group, a dodecamethylene group, a tridecamethylene group, a tetradecamethylene group, a hexadecamethylene group, and an octadecamethylene group; and branched alkylene groups such as a propylene group, a 2-methyltetramethylene group, a 2-methylpentamethylene group, a 3-methylpentamethylene group, and a 2-ethyloctamethylene group. Among them, a nonamethylene group, a decamethylene group, an undecamethylene group, a dodecamethylene group, or a tridecamethylene group is preferred.
$R^{20}$ and $R^{21}$ are each independently a hydrogen atom or a linear or branched hydrocarbon group having 1 to 28

carbon atoms, and at least one of them is a hydrogen atom. Examples of the linear or branched hydrocarbon group having 1 to 28 carbon atoms include: linear alkyl groups such as a methyl group, an ethyl group, a propyl group, a butyl group, a pentyl group, a hexyl group, an octyl group, a decyl group, a dodecyl group, a tetradecyl group, a hexadecyl group, an octadecyl group, an eicosyl group, a doeicosyl group, a tetraeicosyl group, a hexaeicosyl group, and an octaeicosyl group; and branched alkyl groups such as an isopropyl group, a sec-butyl group, a tert-butyl group, a neopentyl group, a 1-ethylpropyl group, and a 1-heptyldecyl group. Among them, preferably, both of $R^{20}$ and $R^{21}$ are hydrogen atoms.

In the formula (2), p represents the number of repeat units and represents a number of from 5 to 60, preferably a number of from 20 to 30, as an average value. In this context, the average value of p is calculated from the intensity ratio between peaks attributed to $R^{14}$ and $R^{15}$ by $^1$H-NMR on the basis of terminal methyl groups of hydrocarbon groups introduced to both ends of polysiloxane.

q represents the number of repeat units and represents a number of from 2.5 to 10, preferably a number of from 3.0 to 6.0, as an average value. In this context, the average value of q is calculated from the intensity ratio between peaks attributed to methine and methylene hydrogens attached by $OR^{20}$ and $OR^{21}$ by $^1$H-NMR on the basis of terminal methyl groups of hydrocarbon groups introduced to both ends of polysiloxane.

The constituent units with the numbers of repeats, p and q, may be block copolymers or random copolymers and are preferably random copolymers. Preferably, p is larger than q.

The alkyl glyceryl ether-modified silicone of the formula (2) can be produced in accordance with a method described in, for example, JP-A-4-134013, through the hydrosilylation reaction of organo hydrogen polysiloxane having at least one silicon-hydrogen bond with corresponding alkenyl glyceryl ether or the like.

The alkyl glyceryl ether-modified silicone of the formula (2) is preferably glyceryl undecyl dimethicone or bisalkyl (C16-18) glyceryl undecyl dimethicone from the viewpoint of forming a network structure between moisture present on the skin and the ester oil as the component (D) during or after application so that a coating film unlikely to cause makeup wear-off is formed on the skin, thereby preventing makeup wear-off, shine ascribable to sebum, and the like, improving makeup durability, and also improving preservation stability at high temperatures.

One of or two or more in combination of these components (C) can be used. The content is preferably 0.1% by mass or more, more preferably 1% by mass or more, further more preferably 2% by mass or more, and preferably 12% by mass or less, more preferably 7% by mass or less, further more preferably 5% by mass or less, in the entire composition of the water-in-oil emulsion composition from the viewpoint of the prevention of makeup wear-off, shine ascribable to sebum, and the like, and improvement in makeup durability. The content of the component (C) is preferably from 0.1 to 12% by mass, more preferably from 1 to 7% by mass, further more preferably from 2 to 5% by mass, in the entire composition of the water-in-oil emulsion composition.

In the present invention, the mass ratio of the component (A) to the component (C), (A)/(C), is preferably 0.05 or more, more preferably 0.1 or more, further more preferably 0.2 or more, and preferably 3 or less, more preferably 1 or less, further more preferably 0.6 or less, from the viewpoint of improvement in adhesiveness to the skin, favorable fit feeling to the skin, the absence of makeup wear-off even after a lapse of a long time, excellent makeup durability, and also excellent emulsification stability at high temperatures. The mass ratio of the component (A) to the component (C), (A)/(C), is preferably from 0.05 to 3, more preferably from 0.1 to 1, further more preferably from 0.2 to 0.6.

In the present invention, the mass ratio of the component (B) to the component (C), (B)/(C), is preferably 0.5 or more, more preferably 1 or more, further more preferably 2.5 or more, and preferably 15 or less, more preferably 7 or less, further more preferably 5.5 or less, from the viewpoint of improvement in adhesiveness to the skin, favorable fit feeling to the skin, the absence of makeup wear-off even after a lapse of a long time, excellent makeup durability, and also excellent emulsification stability at high temperatures. The mass ratio of the component (B) to the component (C), (B)/(C), is preferably from 0.5 to 15, more preferably from 1 to 7, further more preferably from 2.5 to 5.5.

The component (D) is an ester oil which is in a liquid state at 25°C.

In this context, the liquid state refers to having a viscosity of 10 000 mPa·s or less at 25°C, as in the component (B). The viscosity is measured by the same method as in the component (B).

The ester oil as the component (D) may not be limited as long as it can be used in usual cosmetics. Examples thereof include: monoester oils such as ethylhexyl p-methoxycinnamate, cetyl ethylhexanoate, octyldodecyl myristate, isotridecyl isononanoate, and isononyl isononanoate; diester oils such as neopentyl glycol dicaprate, propylene glycol dicaprate, and diisostearyl malate; triester oils such as glyceryl tri-2-ethylhexanoate and glyceryl tri(caprylate/caprate); and tetraester oils such as dipentaerythrityl tetraisostearate.

Among them, an ester oil having a molecular weight of 300 or larger is preferred from the viewpoint of improvement in adhesiveness to the skin, favorable fit feeling to the skin, the absence of makeup wear-off even after a lapse of a long time, and excellent makeup durability. The ester oil preferably comprises one or more members selected from the group consisting of monoester and diester, more preferably comprises one or more members selected from the group consisting of diisostearyl malate, neopentyl glycol dicaprate, isotridecyl isononanoate, cetyl ethylhexanoate, and propylene glycol dicaprate, and further more preferably comprise at least one or two members selected from the group consisting of diisostearyl malate and neopentyl glycol dicaprate.

One of or two or more in combination of these components (D) can be used. The content is preferably 3% by mass or more, more preferably 6% by mass or more, further more preferably 10% by mass or more, and preferably 40% by mass or less, more preferably 25% by mass or less, further more preferably 16% by mass or less, in the entire composition of the water-in-oil emulsion composition from the viewpoint of improvement in adhesiveness to the skin, favorable fit feeling to the skin, the absence of makeup wear-off even after a lapse of a long time, and excellent makeup durability. The content of the component (D) is preferably from 3 to 40% by mass, more preferably from 6 to 25% by mass, further more preferably from 10 to 16% by mass, in the entire composition of the water-in-oil emulsion composition.

In the present invention, the mass ratio of the component (B) to the component (D), (B)/(D), is preferably 0.2 or more, more preferably 0.4 or more, further more preferably 0.6 or more, and preferably 5 or less, more preferably 3 or less, further more preferably 1.5 or less, from the viewpoint of improvement in adhesiveness to the skin, favorable fit feeling to the skin, the absence of makeup wear-off even after a lapse of a long time, excellent makeup durability, and also excellent emulsification stability at high temperatures. The mass ratio of the component (B) to the component (D), (B)/(D), is preferably from 0.2 to 5, more preferably from 0.4 to 3, further more preferably from 0.6 to 1.5.

The water-in-oil emulsion composition of the present invention can further comprise a hydrophobized color pigment from the viewpoint of improving finish coverage and beautiful makeup finish feeling of the whole face.

The color pigment is a color pigment for use in usual cosmetics. Examples thereof include: inorganic pigments including metal oxides such as titanium oxide, zinc oxide, cerium oxide, aluminum oxide, yellow iron oxide, black iron oxide, red iron oxide, iron blue, ultramarine, chromium oxide, and chromium hydroxide, metal complexes such as manganese violet and cobalt titanate, and further, carbon black; synthetic organic pigments, organic dyes, and lake pigments thereof, such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 405, Red No. 505, Orange No. 203, Orange No. 204, Orange No. 205, Yellow No. 4, Yellow No. 5, Yellow No. 401, Blue No. 1, and Blue No. 404; and natural organic dyes such as β-carotene, caramel, and paprika pigment, and include complexes of these color pigments, and mixed pigments of these color pigments diluted with extenders such as barium sulfate or talc.

The color pigment preferably contains a metal oxide, more preferably comprises one or more members selected from the group consisting of titanium oxide, zinc oxide, yellow iron oxide, black iron oxide, and red iron oxide, and further more preferably comprises one or more members selected from the group consisting of titanium oxide, yellow iron oxide, black iron oxide, and red iron oxide, from the viewpoint of covering the skin and imparting aesthetic makeup effects.

The hydrophobization treatment of such a color pigment is not limited as long as the treatment can be practiced for usual cosmetic powders. Dry treatment, wet treatment, or the like can be performed using a surface treatment agent such as a silicone compound, a metal soap, an amino acid compound, lecithin, an oil agent, or organic titanate.

Specific examples of the surface treatment agent include: silicone compounds including alkylalkoxysilane such as methyltrimethoxysilane, ethyltrimethoxysilane, hexyltrimethoxysilane, octyltrimethoxysilane, octyltriethoxysilane, and triethoxycaprylylsilane, dimethylpolysiloxane, methyl hydrogen polysiloxane, cyclic silicone, cross-linked silicone, silicone resin, and acrylic modified silicone; metal soaps such as aluminum stearate, aluminum myristate, zinc stearate, and magnesium stearate; amino acids such as proline, hydroxyproline, alanine, glycine, sarcosine, glutamic acid, aspartic acid, lysine, and their derivatives; acylated amino acids such as stearoyl glutamic acid, lauroyl aspartic acid, dilauramidoglutamide lysine, and lauroyl lysine; lecithin and hydrogenated lecithin; oil agents such as polyisobutylene, waxes, fat/oil, and fatty acids; and organic titanate such as isopropyl triisostearoyl titanate.

The acylated amino acid includes salts of the acylated amino acid. Examples of the salt of the acylated amino acid include Na, Ca, Al, Mg, Zn, Zr, and Ti salts. The salt of the acylated amino acid preferably comprises one or more members selected from the group consisting of Na and Ca salts, and more preferably comprises Na salt. The acylated amino acid salt preferably comprises one or more members selected from the group consisting of disodium stearoyl glutamate and sodium lauroyl aspartate, and more preferably comprises disodium stearoyl glutamate, from the viewpoint of improvement in adhesiveness to the skin, favorable fit feeling to the skin, excellent makeup durability, and excellent stability at high temperatures.

The surface treatment agent preferably comprises one or more members selected from the group consisting of a silicone compound, an acylated amino acid, and organic titanate, more preferably comprises one or more members selected from the group consisting of dimethylpolysiloxane, methyl hydrogen polysiloxane, alkylalkoxysilane, disodium stearoyl glutamate, sodium lauroyl aspartate, and isopropyl triisostearoyl titanate, further more preferably comprises one or more members selected from the group consisting of dimethylpolysiloxane, alkylalkoxysilane, disodium stearoyl glutamate, and isopropyl triisostearoyl titanate, is even more preferably disodium stearoyl glutamate, from the viewpoint of improvement in adhesiveness to the skin, favorable fit feeling to the skin, excellent makeup durability, and excellent stability at high temperatures.

The hydrophobization treatment can be performed in accordance with a usual method.

The amount of the treatment agent in the hydrophobization treatment is preferably from 0.1 to 15% by mass, more preferably from 1 to 12% by mass, based on the powder to be coated from the viewpoint of improvement in dispersibility in a solvent.

One or more of these hydrophobized color pigments can be used. The content is preferably 1% by mass or more, more preferably 3% by mass or more, further more preferably 5% by mass or more, and preferably 30% by mass or less, more preferably 25% by mass or less, further more preferably 20% by mass or less, in the entire composition of the water-in-oil emulsion composition from the viewpoint of improvement in adhesiveness to the skin, favorable fit feeling to the skin, excellent makeup durability, and excellent stability at high temperatures. The content of the hydrophobized color pigment is preferably from 1 to 30% by mass, more preferably from 3 to 25% by mass, further more preferably from 5 to 20% by mass, in the entire composition of the water-in-oil emulsion composition.

The water-in-oil emulsion composition of the present invention can further comprise a volatile oil.

The term "volatile" refers to having a flash point of from -4 to 88°C. Examples of the volatile oil include volatile silicone oils and volatile hydrocarbon oils.

Examples of the volatile silicone oil include: linear dimethylpolysiloxane such as hexamethyldisiloxane, octamethyltrisiloxane (1 cs), decamethyltetrasiloxane (1.5 cs), and dimethylpolysiloxane (2 cs); branched siloxane such as methyl trimethicone, tris(trimethylsilyl)methylsilane, and tetrakis(trimethylsilyl)silane; and cyclic dimethylsiloxane such as octamethylcyclotetrasiloxane, decamethylcyclopentasiloxane, and dodecamethylcyclohexasiloxane.

Examples of the volatile hydrocarbon oil include: paraffin hydrocarbon oils such as n-decane, n-undecane, and n-dodecane; isoparaffin hydrocarbon oils such as isodecane, isododecane, and hydrogenated polyisobutene; and cyclic paraffin hydrocarbon oils such as cyclodecane and cyclododecane. Among them, a hydrocarbon oil having 8 to 16 carbon atoms is preferred, a hydrocarbon oil having 10 to 16 carbon atoms is more preferred, and a hydrocarbon oil having 12 carbon atoms is further more preferred.

The volatile oil preferably is one or more members selected from the group consisting of a hydrocarbon oil and a silicone oil, and preferably comprises one or more members selected from the group consisting of isododecane, hexamethyldisiloxane, methyl trimethicone, and dimethylpolysiloxane having a kinetic viscosity of 2 cSt or less at 25°C, from the viewpoint of forming a uniform coating film on the skin, improving adhesiveness, and improving fit feeling to the skin. The kinetic viscosity can be measured using, for example, an Ubbelohde viscometer.

Examples of commercially available products of the hexamethyldisiloxane and the dimethylpolysiloxane having a kinetic viscosity of 2 cSt or less at 25°C include "KF-96L-0.65 cs" (hexamethyldisiloxane), "TMF1.5", "KF-96L-1 cs" (octamethyltrisiloxane), "KF-96L-1.5 cs", and "KF-96L-2 cs" manufactured by Shin-Etsu Chemical Co., Ltd., "SH200C Fluid 1 cs" and "SH200C Fluid 1.5 cs" manufactured by DuPont Toray Specialty Materials K.K., "TSF451-0.65" manufactured by Momentive Performance Materials Japan LLC, "BELSIL DM0.65" manufactured by Wacker Asahikasei Silicone Co., Ltd., and "XIAMETER PMX-200 SILICONE FLUID 0.65 CS" manufactured by Dow Toray Co., Ltd.

One of or two or more in combination of these volatile oils can be used. The content is preferably 1% by mass or more, more preferably 4% by mass or more, further more preferably 8% by mass or more, and preferably 50% by mass or less, more preferably 35% by mass or less, further more preferably 25% by mass or less, in the entire composition of the water-in-oil emulsion composition from the viewpoint of forming a uniform coating film on the skin, improving adhesiveness, and improving fit feeling to the skin. The content of the volatile oil is preferably from 1 to 50% by mass, more preferably from 4 to 35% by mass, further more preferably from 8 to 25% by mass, in the entire composition of the water-in-oil emulsion composition.

The water-in-oil emulsion composition of the present invention preferably has a content of an organic resin powder such as microplastics of 1% by mass or less, more preferably 0.1% by mass or less, in the entire composition, and further more preferably is substantially free from an organic resin powder, from the viewpoint of environmental friendliness, excellent use impression, and excellent stability at high temperatures.

Specifically, the organic resin powder refers to other than a cellulose powder excellent in biodegradability.

Examples of the organic resin powder include: polyamide resin, nylon resin, polyester resin, polyethylene resin, polyethylene tetrafluoride resin, polypropylene resin, polystyrene resin, benzoguanamine resin, polymethylbenzoguanamine resin, polyurethane resin, vinyl resin, fluorine resin, acrylic resin, and melamine resin; cross-linked or non-cross-linked organic resin powders such as powders of one or more polymers or copolymers selected from the group consisting of butyl acrylate-vinyl acetate copolymers, styrene-acrylic acid copolymers, silicone resin, and divinylbenzene-styrene copolymers.

In the water-in-oil emulsion composition of the present invention, the content of water is preferably from 3 to 50% by mass, more preferably from 5 to 40% by mass, further more preferably from 10 to 30% by mass, in the entire composition from the viewpoint of the formation of a stable emulsion composition.

In the water-in-oil emulsion composition of the present invention, various components usually used in cosmetics can be optionally used, in addition to the components described above. Examples of these components include oil components other than those described above, surfactants, water-soluble polymers, antioxidants, ultraviolet absorbers, vitamins, antiseptics, pH adjusters, fragrances, plant extracts, humectants, colorants, cooling agents, antiperspirants, germicides, and skin activators.

The water-in-oil emulsion composition of the present invention can be produced in accordance with a usual method and can be prepared in a dosage form such as a liquid, emulsion, paste, cream, gel, or solid form.

The water-in-oil emulsion composition of the present invention can be prepared into a water-in-oil emulsion cosmetic and can be applied as, for example, a makeup cosmetic such as a makeup base, a foundation, a concealer, a blusher, an eye shadow, a mascara, an eyeliner, an eyebrow liner, an overcoat, or a lipstick; an ultraviolet protection cosmetic such as a sunscreen emulsion or a sunscreen cream; or a skincare cosmetic such as a skincare emulsion, a skincare cream, a BB cream, or a serum.

Among them, the water-in-oil emulsion composition of the present invention is suitable as a water-in-oil emulsion cosmetic in a liquid state.

The viscosity of the water-in-oil emulsion composition of the present invention at 30°C is preferably from 3 000 to 30 000 mPa·s, more preferably from 5 000 to 25 000 mPa·s, from the viewpoint of forming a uniform coating film on the skin, improving adhesiveness, and improving fit feeling to the skin.

The viscosity was measured using a B type viscometer (manufactured by Toki Sangyo Co., Ltd., TVB-10 model) under conditions involving the number of rotations of 12 rpm, a measurement time of 30 seconds, and a temperature of 30°C. The rotors used were the following rotors depending on the viscosity of the composition.

Viscosity of 2 500 mPa·s or more and less than 10 000 mPa·s: rotor No. 3, and
Viscosity of 10 000 mPa·s or more: rotor No. 4

In Table 1, a water-in-oil emulsion composition of Example 1 had a viscosity of 12 000 mPa·s at 30°C.

The water-in-oil emulsion composition of the present invention can be used as a water-in-oil emulsion composition for impregnation by impregnating the composition into a holder and taking the composition by the finger or an applicator from the holder to apply the composition to the skin.

The holder is a material which holds the water-in-oil emulsion composition having flowability by impregnation. Examples thereof include nonwoven fabrics made of a single or mixed material such as resin, pulp, and cotton, resonated fiber materials, foams such as sponge, porous materials having a continuous mechanism, and assemblies of fibers.

Examples of the material include NBR (acrylonitrile-butadiene rubber), SBR (styrene-butadiene rubber), NR (natural rubber), urethane, nylon, polyolefin, polyester, EVA (ethylene vinyl acetate), PVA (polyvinyl alcohol), and silicone elastomers. Any material may be used as long as the holder can have contents. Among them, an assembly of polyester fibers is preferred. It is more preferred to use a fiber material formed using polyester fibers with a hardness of preferably from 40 to 60 (value measured with an F durometer) and an apparent density (which abides by JIS K 6400-1) of preferably from 0.006 to 0.1 g/cm$^3$, more preferably from 0.02 to 0.1 g/cm$^3$, which may be an assembly of polyester fibers having a layer structure where front and back layers are connected through cross-linked threads.

Among them, examples of the assembly of fibers include assemblies of fibers having a core-sheath structure. The fibers having a core-sheath structure are fibers having an internal core moiety surrounded by a sheath moiety. Preferably, the fibers are made of a thermoplastic resin, and the core moiety is constituted by a thermoplastic resin having a higher melting point than that of the sheath moiety. Materials for the core moiety and the sheath moiety in the fibers having a core-sheath structure are preferably polyolefin resin which is free from time-dependent deterioration ascribable to the components of the emulsion composition, though they are not particularly limited.

The polyolefin resin which can be used has the core moiety constituted by polypropylene (melting point: 165°C) and the sheath moiety constituted by polyethylene (melting point: 130°C), or has the core moiety constituted by polypropylene (melting point: 165°C) and the sheath moiety constituted by polybutene (melting point: 127°C). The fibers having a core-sheath structure preferably have a thickness of from 2.0 to 15 dtex, more preferably from 2.0 to 13 dtex, from the viewpoint of holding the emulsion composition by impregnation.

Eccentric core-sheath composite fibers are preferred for three-dimensional crimps. The shape of the core moiety in the eccentric core-sheath type may be a circle as well as a variant shape such as an oval, Y, or X shape, and the sectional shape of the eccentric core-sheath fibers may also be a circle as well as a variant shape such as an oval, Y, X, hash mark, polygonal, or star shape, or a hollow shape.

The assembly of the fibers having a core-sheath structure which can be used can be produced by a known method. Examples thereof include a thermal bond method, a needle punch method, and a spun lacing method. The assembly of the fibers having a core-sheath structure more preferably has partially fused sheath moieties in the fibers having a core-sheath structure. The assembly of the fibers having a core-sheath structure is not limited by its production method, and the method can be performed by, for example, a blast conveyor furnace scheme, a molding scheme, or a method using a mesh belt (method described in JP-B-4195043). Then, a size suitable for the holder can be created by cutting treatment such as pressing, though the method is not limited thereto.

The assembly of the fibers having a core-sheath structure more preferably has an apparent density (which abides by JIS K 6400-1) of from 0.02 to 0.1 g/cm$^3$ from the viewpoint of the holdability of the emulsion composition by impregnation.

The size of the assembly of the fibers having a core-sheath structure is not particularly limited and can be determined depending on, for example, easy use or the size of a container or the like in which it is to be housed.

The holder may be in a state wholly or partially impregnated with the water-in-oil emulsion composition or may be disposed as a screen net on the surface of the water-in-oil emulsion composition.

The weaving form of the screen net is not limited by its yarn texture configuration method, yarn shape, yarn twist count, thickness, material, or the like and may be, for example, plain weave, 1/2 twill weave, 2/1 twill weave, twill weave, gauze weave, half-gauze weave, or satin weave.

Examples of the screen net include screen nets made of polyurethane, TPE (thermoplastic plastic elastomer), polyester, polyether, acryl, or olefin. The thickness of the fibers constituting the screen net is preferably from 0.01 to 1.0 mm.

It is preferred that the screen net should be completely attached to the holder impregnated with the emulsion composition by tight contact, or an absorber should be positioned 0.1 to 3.0 mm distant from the screen net without adhesion between the absorber and the screen net.

The water-in-oil emulsion composition of the present invention can be used by impregnating the composition into such a holder and taking the composition by the finger or an applicator from the holder to apply the composition to the skin.

The water-in-oil emulsion composition of the present invention can be used by impregnating such a holder therewith, positioning a screen net adjacently therewith, and using the finger or an applicator to apply the composition from the screen net to the skin.

Examples of the applicator for application to the skin include sponge, puff, and chips which are usually used in applying liquid cosmetics to the skin.

The water-in-oil emulsion composition of the present invention can be prepared into a cosmetic in which a holder impregnated with the water-in-oil emulsion composition is housed in a compact container.

The water-in-oil emulsion composition of the present invention can be prepared into a cosmetic in which a holder impregnated with the water-in-oil emulsion composition and a screen net are positioned adjacently with each other and are housed in a compact container.

The water-in-oil emulsion composition of the present invention can be prepared into a cosmetic kit comprising a set of a holder impregnated with the water-in-oil emulsion composition, and a compact container.

The water-in-oil emulsion composition of the present invention can be used by the impregnation of a holder and, in addition, may be suitably used as a usual liquid water-in-oil emulsion cosmetic, particularly, a liquid foundation, without impregnating the holder therewith.

In relation to the embodiments mentioned above, the present invention further discloses the following compositions and the like.

<1> A water-in-oil emulsion composition for impregnation comprising the following components (A), (B), (C) and (D):

(A) cation-modified clay mineral substituted with a quaternary ammonium ion,
(B) one or more members selected from the group consisting of phenyl-modified silicone and alkyl-modified silicone which are in a liquid state at 25°C,
(C) modified silicone containing an alkyl group having 10 or more carbon atoms and a polyglyceryl group, and
(D) an ester oil which is in a liquid state at 25°C.

<2> A cosmetic in which a holder impregnated with a water-in-oil emulsion composition is housed in a compact container, the water-in-oil emulsion composition comprising the following components (A), (B), (C) and (D):

(A) cation-modified clay mineral substituted with a quaternary ammonium ion,
(B) one or more members selected from the group consisting of phenyl-modified silicone and alkyl-modified silicone which are in a liquid state at 25°C,
(C) modified silicone containing an alkyl group having 10 or more carbon atoms and a polyglyceryl group, and
(D) an ester oil which is in a liquid state at 25°C.

<3> The cosmetic according to <2>, wherein the component (A) is preferably cation-modified clay mineral obtained by substituting layered clay mineral by a quaternary ammonium salt cationic surfactant, more preferably cation-modified clay mineral obtained by substituting layered clay mineral by a quaternary ammonium salt cationic surfactant of the following formula (1):

$$(R^1R^2R^3R^4N)^+X^- \qquad (1)$$

wherein $R^1$ represents an alkyl group having 10 to 22 carbon atoms or a benzyl group, $R^2$ represents a methyl group or an alkyl group having 10 to 22 carbon atoms, each of $R^3$ and $R^4$ represents an alkyl group or a hydroxyalkyl group having 1 to 3 carbon atoms, and X represents a halogen atom or a methyl sulfate residue, further more preferably

comprises one or more members selected from the group consisting of dimethyldistearyl ammonium hectorite, dimethyldistearyl ammonium bentonite, and benzyldimethylstearyl ammonium hectorite, even more preferably comprises one or more members selected from the group consisting of dimethyldistearyl ammonium hectorite and benzyldimethylstearyl ammonium hectorite, and even more preferably comprises dimethyldistearyl ammonium hectorite.

<4> The cosmetic according to <2> or <3>, wherein a content of the component (A) is preferably 0.1% by mass or more, more preferably 0.3% by mass or more, further more preferably 0.5% by mass or more, and preferably 8% by mass or less, more preferably 4% by mass or less, further more preferably 2% by mass or less, in terms of a solid content in the entire composition of the water-in-oil emulsion composition.

<5> The cosmetic according to any one of <2> to <4>, wherein the phenyl-modified silicone which is in a liquid state at 25°C as the component (B) is preferably methylphenylpolysiloxane, phenyl trimethicone, diphenylsiloxyphenyl trimethicone, diphenyl dimethicone, trimethylsiloxyphenyl dimethicone, or trimethylpentaphenyltrisiloxane, more preferably comprises one or more members selected from the group consisting of methylphenylpolysiloxane, phenyl trimethicone, and diphenylsiloxyphenyl trimethicone, and further more preferably comprises at least methylphenylpolysiloxane.

<6> The cosmetic according to any one of <2> to <4>, wherein the alkyl-modified silicone which is in a liquid state at 25°C as the component (B) is preferably caprylyl methicone, cetyl dimethicone, alkyl (C26-28) dimethicone, stearyl dimethicone, behenoxy dimethicone, or an (acrylates/stearyl acrylate/dimethicone methacrylate) copolymer, more preferably comprises at least one or more members selected from the group consisting of caprylyl methicone, cetyl dimethicone, alkyl (C26-28) dimethicone, stearyl dimethicone, and behenoxy dimethicone, and further more preferably comprises at least caprylyl methicone.

<7> The cosmetic according to any one of <2> to <6>, wherein the phenyl-modified silicone and the alkyl-modified silicone which are in a liquid state at 25°C are preferably used in combination as the component (B).

<8> The cosmetic according to any one of <2> to <7>, wherein a content of the component (B) is preferably 1% by mass or more, more preferably 6% by mass or more, further more preferably 11% by mass or more, and preferably 30% by mass or less, more preferably 21% by mass or less, further more preferably 16% by mass or less, in the entire composition of the water-in-oil emulsion composition.

<9> The cosmetic according to any one of <2> to <8>, wherein the component (C) preferably has HLB of from 2 to 7, more preferably HLB of from 2 to 5.

<10> The cosmetic according to any one of <2> to <9>, wherein the component (C) preferably comprises both-terminally alkyl-modified glyceryl ether silicone.

<11> The cosmetic according to any one of <2> to <10>, wherein the component (C) preferably comprises at least one or more members selected from the group consisting of lauryl PEG-10 tris(trimethylsiloxy)silylethyl dimethicone, cetyl diglyceryl tris(trimethylsiloxy)silylethyl dimethicone, cetyl PEG/PPG-10/1 dimethicone, lauryl PEG-9 polydimethylsiloxyethyl dimethicone, and lauryl PEG/PPG-18/18 methicone.

<12> The cosmetic according to any one of <2> to <9>, wherein the component (C) preferably comprises alkyl glyceryl ether-modified silicone of the formula (2):

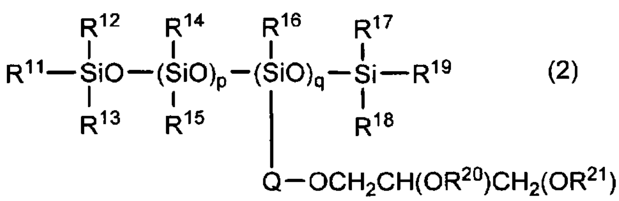

(2)

wherein $R^{11}$ and $R^{19}$ are the same or different and are each independently a linear or branched hydrocarbon group having 1 to 32 carbon atoms in terms of a mode value; $R^{12}$ to $R^{18}$ are the same or different and are each independently a linear or branched hydrocarbon group having 1 to 5 carbon atoms; Q is a linear or branched divalent hydrocarbon group having 3 to 20 carbon atoms; $R^{20}$ and $R^{21}$ are each independently a hydrogen atom or a linear or branched hydrocarbon group having 1 to 28 carbon atoms, and at least one of them is a hydrogen atom; p represents the number of repeat units and is a number of 5 or more and 60 or less as an average value; q represents the number of repeat units and is a number of 2.5 or more and 10 or less as an average value; and constituent units with the number of repeats, p and q, are any of block copolymers and random copolymers, provided that the formula has one or more alkyl groups having 10 or more carbon atoms,
more preferably glyceryl undecyl dimethicone or bisalkyl (C16-18) glyceryl undecyl dimethicone.

<13> The cosmetic according to any one of <2> to <12>, wherein a content of the component (C) is preferably 0.1% by

mass or more, more preferably 1% by mass or more, further more preferably 2% by mass or more, and preferably 12% by mass or less, more preferably 7% by mass or less, further more preferably 5% by mass or less, in the entire composition of the water-in-oil emulsion composition.

<14> The cosmetic according to any one of <2> to <13>, wherein a mass ratio of the component (A) to the component (C), (A)/(C), is preferably 0.05 or more, more preferably 0.1 or more, further more preferably 0.2 or more, and preferably 3 or less, more preferably 1 or less, further more preferably 0.6 or less.

<15> The cosmetic according to any one of <2> to <14>, wherein a mass ratio of the component (B) to the component (C), (B)/(C), is preferably 0.5 or more, more preferably 1 or more, further more preferably 2.5 or more, and preferably 15 or less, more preferably 7 or less, further more preferably 5.5 or less.

<16> The cosmetic according to any one of <2> to <15>, wherein the component (D) is preferably an ester oil having a molecular weight of 300 or larger, more preferably comprises one or more members selected from the group consisting of monoester and diester, further more preferably comprises one or more members selected from the group consisting of diisostearyl malate, neopentyl glycol dicaprate, isotridecyl isononanoate, cetyl ethylhexanoate, and propylene glycol dicaprate, and even more preferably comprises at least one or two members selected from the group consisting of diisostearyl malate and neopentyl glycol dicaprate.

<17> The cosmetic according to any one of <2> to <16>, wherein a content of the component (D) is preferably 3% by mass or more, more preferably 6% by mass or more, further more preferably 10% by mass or more, and preferably 40% by mass or less, more preferably 25% by mass or less, further more preferably 16% by mass or less, in the entire composition of the water-in-oil emulsion composition.

<18> The cosmetic according to any one of <2> to <17>, wherein a mass ratio of the component (B) to the component (D), (B)/(D), is preferably 0.2 or more, more preferably 0.4 or more, further more preferably 0.6 or more, and preferably 5 or less, more preferably 3 or less, further more preferably 1.5 or less.

<19> The cosmetic according to any one of <2> to <18>, wherein preferably, a content of the component (A) is from 0.1 to 8% by mass, a content of the component (B) is from 1 to 30% by mass, a content of the component (C) is from 0.1 to 12% by mass, and a content of the component (D) is from 3 to 40% by mass.

<20> The cosmetic according to any one of <2> to <19>, wherein the water-in-oil emulsion composition preferably further comprises a hydrophobized color pigment, and more preferably comprises 1 to 30% by mass of a hydrophobized color pigment in the entire composition of the water-in-oil emulsion composition.

<21> The cosmetic according to <20>, wherein the color pigment preferably comprises metal oxide, more preferably comprises one or more members selected from the group consisting of titanium oxide, zinc oxide, yellow iron oxide, black iron oxide, and red iron oxide, and further more preferably comprises one or more members selected from the group consisting of titanium oxide, yellow iron oxide, black iron oxide, and red iron oxide.

<22> The cosmetic according to <20> or <21>, wherein a surface treatment agent for the hydrophobization treatment preferably comprises one or more members selected from the group consisting of a silicone compound, an acylated amino acid, and organic titanate, more preferably comprises one or more members selected from the group consisting of dimethylpolysiloxane, methyl hydrogen polysiloxane, alkylalkoxysilane, disodium stearoyl glutamate, sodium lauroyl aspartate, and isopropyl triisostearoyl titanate, further more preferably comprises one or more members selected from the group consisting of dimethylpolysiloxane, alkylalkoxysilane, disodium stearoyl glutamate, and isopropyl triisostearoyl titanate, and is even more preferably disodium stearoyl glutamate.

<23> The cosmetic according to any one of <20> to <22>, wherein a content of the hydrophobized color pigment is preferably 1% by mass or more, more preferably 3% by mass or more, further more preferably 5% by mass or more, and preferably 30% by mass or less, more preferably 25% by mass or less, further more preferably 20% by mass or less, in the entire composition of the water-in-oil emulsion composition.

<24> The cosmetic according to any one of <2> to <23>, wherein the water-in-oil emulsion composition preferably further comprises a volatile oil, and more preferably comprises 1 to 50% by mass of the volatile oil in the entire composition of the water-in-oil emulsion composition.

<25> The cosmetic according to <24>, wherein the volatile oil is preferably one or more members selected from the group consisting of a volatile hydrocarbon oil and a volatile silicone oil, and more preferably comprises one or more members selected from the group consisting of isododecane, hexamethyldisiloxane, methyl trimethicone, and dimethylpolysiloxane having a kinetic viscosity of 2 cSt or less at 25°C.

<26> The cosmetic according to <24> or <25>, wherein a content of the volatile oil is preferably 1% by mass or more, more preferably 4% by mass or more, further more preferably 8% by mass or more, and preferably 50% by mass or less, more preferably 35% by mass or less, further more preferably 25% by mass or less, in the entire composition of the water-in-oil emulsion composition.

<27> The cosmetic according to any one of <2> to <26>, wherein the water-in-oil emulsion composition preferably has an organic resin powder content of 1% by mass or less, more preferably 0.1% by mass or less, in the entire composition, and further more preferably is substantially free from an organic resin powder.

<28> The cosmetic according to any one of <2> to <27>, wherein a content of water in the water-in-oil emulsion

composition is preferably from 3 to 50% by mass, more preferably from 5 to 40% by mass, further more preferably from 10 to 30% by mass, in the entire composition.

<29> The cosmetic according to any one of <2> to <28>, wherein the water-in-oil emulsion composition is preferably a water-in-oil emulsion cosmetic in a liquid state.

<30> The cosmetic according to any one of <2> to <29>, wherein a viscosity at 30°C of the water-in-oil emulsion composition is preferably from 3 000 to 30 000 mPa·s, more preferably from 5 000 to 25 000 mPa·s.

<31> The cosmetic according to any one of <2> to <30>, wherein the holder is preferably a foam, a porous material, or an assembly of fibers, more preferably an assembly of polyester fibers.

<32> A method for using a cosmetic according to any one of <2> to <31>, comprising taking a water-in-oil emulsion composition impregnated into a holder, by finger or an applicator from the holder, and applying it to the skin for use.

<33> A cosmetic kit comprising a set of a holder impregnated with a water-in-oil emulsion composition, and a compact container, the water-in-oil emulsion composition comprising the following components (A), (B), (C) and (D):

(A) cation-modified clay mineral substituted with a quaternary ammonium ion,
(B) one or more members selected from the group consisting of phenyl-modified silicone and alkyl-modified silicone which are in a liquid state at 25°C,
(C) modified silicone containing an alkyl group having 10 or more carbon atoms and a polyglyceryl group, and
(D) an ester oil which is in a liquid state at 25°C.

<34> A water-in-oil emulsion composition comprising the following components (A), (B), (C) and (D):

(A) cation-modified clay mineral substituted with a quaternary ammonium ion,
(B) one or more members selected from the group consisting of phenyl-modified silicone and alkyl-modified silicone which are in a liquid state at 25°C,
(C) modified silicone containing an alkyl group having 10 or more carbon atoms and a polyglyceryl group, and
(D) an ester oil which is in a liquid state at 25°C.

Examples

Production Example 1 (Production of both terminally alkyl (C16-18)-modified dimethylsiloxane-methyl(undecyl glyceryl ether)siloxane copolymer):

(1) Step-1 (Synthesis of tetramethyldisiloxane having, at both ends of silicone chain, alkyl groups different from other alkyl groups in the silicone chain):

44.8 g of 1,1,3,3-tetramethyldisiloxane and 1.0 g of Spier's catalyst (solution containing 2% by mass of chloroplatinic acid in 2-propanol) were added to a three-neck flask and warmed to 70°C. In a nitrogen atmosphere, 174.2 g of $\alpha$-olefin (manufactured by Idemitsu Kosan Co., Ltd., "LINEAREN 168", a 57/43 (mass ratio) mixture of olefins having 16 and 18 carbon atoms)) was added dropwise thereto at 70°C, followed by stirring for 2 hours. After cooling, the reaction system was neutralized with an aqueous sodium hydroxide solution, and distillation and purification were performed under reduced pressure. The obtained product was confirmed from the $^1$H-NMR spectrum (400 MHz) of the obtained product to be a 1,1,3,3-tetramethyldisiloxane derivative having alkyl groups having 16 carbon atoms and having 18 carbon atoms at both ends (22.1 g, yield; 85%).

(2) Step-2 (Synthesis of organo hydrogen polysiloxane having, at both ends of silicone chain, alkyl groups different from other alkyl groups in the silicone chain, and having silicon-hydrogen bond in the silicone chain):

44.8 g of the 1,1,3,3-tetramethyldisiloxane derivative having alkyl groups having 16 carbon atoms and having 18 carbon atoms at both ends synthesized in (1), 78.6 g of decamethylcyclopentasiloxane, 19.8 g of 1,3,5,7-tetramethylcyclotetrasiloxane, 50 g of n-heptane, and 5 g of activated earth were added to a three-neck flask and refluxed for 12 hours. After cooling, distillation and purification were performed under reduced pressure. The obtained product was confirmed from the $^1$H-NMR spectrum of the obtained product to be a dimethylsiloxane/methylsiloxane copolymer (p = 23, q = 4) having alkyl groups having 16 carbon atoms and having 18 carbon atoms at both ends (132.8 g, yield; 95%).

(3) Step-3 (Synthesis of polysiloxane both terminally substituted with alkyl groups and modified at side chain with grafted alkyl glyceryl ether group):

50.0 g of the dimethylsiloxane/methylsiloxane copolymer having alkyl groups having 16 carbon atoms and

having 18 carbon atoms at both ends synthesized in (2), 61.0 g of 10-undecenyl glyceryl ether, and 0.25 g of 5% by mass of platinum catalyst supported on carbon were added to a three-neck flask and stirred at 70°C for 3 hours. After cooling, distillation and purification were performed under reduced pressure. The obtained product was confirmed from the $^1$H-NMR spectrum of the obtained product to be a dimethylsiloxane-methyl(undecyl glyceryl ether)siloxane copolymer (p = 23, q = 4) having alkyl groups having 16 carbon atoms and having 18 carbon atoms at both ends (63.0 g, yield; 95%).

The obtained copolymer is a random copolymer of the formula (2) as given below.

$R^{11}$ and $R^{19}$ = an alkyl group having 18 carbon atoms in terms of a mode value,
$R^{12}$ to $R^{18}$ = a methyl group,
Q = an alkylene group having 11 carbon atoms,
$R^{20}$ and $R^{21}$ = a hydrogen atom
p (average value) = 23,
q (average value) = 4.0

Examples 1 to 8 and Comparative Example 1

Water-in-oil emulsion cosmetics (liquid foundations) were produced in accordance with the composition shown in Tables 1 and 2.

The obtained water-in-oil emulsion cosmetics were evaluated for their absence of makeup wear-off after 6 hours, fit feeling to the skin, and emulsification stability, after a holder was impregnated with each cosmetic. The results are also shown in Tables 1 and 2.

(Production method)

A powder component was added to and dispersed in an oil phase component comprising the components (A), (B), (C) and (D). Then, a water phase component was added thereto and stirred with a homomixer to produce a water-in-oil emulsion cosmetic (liquid foundation).

A holder (assembly of polyester fibers) was impregnated with the obtained water-in-oil emulsion cosmetic.

(Evaluation method)

(1) Absence of makeup wear-off after 6 hours

Three expert panelists used puff to apply each water-in-oil emulsion composition (foundation with the holder impregnated therewith) to the skin, and conducted sensory evaluation on the absence of makeup wear-off after a lapse of 6 hours in accordance with criteria given below. The results were indicated by the total score of the three panelists.

5; Makeup wear-off was totally absent.
4; Makeup wear-off was absent.
3; Makeup wear-off was rarely present.
2; Makeup wear-off was slightly present.
1; Makeup wear-off was present.

(2) Fit feeling to skin:

Three expert panelists used puff to apply each water-in-oil emulsion composition (foundation with the holder impregnated therewith) to the skin, and conducted sensory evaluation on the fit feeling to the skin in accordance with criteria given below. The results were indicated by the total score of the three panelists.

5; Adhesiveness to the skin was very good.
4; Adhesiveness to the skin was good.
3; Adhesiveness to the skin was slightly good.
2; Adhesiveness to the skin was not very good.
1; Adhesiveness to the skin was not good.

(3) Emulsification stability:

Each water-in-oil emulsion composition (foundation with the holder impregnated therewith) was preserved for 1

month in an environment of 40°C. Then, its appearance and properties were visually evaluated in accordance with criteria given below.

A; Neither reduction in viscosity nor separation was observed.
B; Viscosity was slightly reduced.
C; Viscosity was slightly reduced, and oil smudge was observed.
D; Separation was observed.

[Table 1]

| | | Component name | Starting material name | Example 1 | Example 2 | Example 3 | Example 4 | Example 5 | Example 6 | Example 7 | Comparative Example 1 |
|---|---|---|---|---|---|---|---|---|---|---|---|
| Oil phase | (A) | Dimethyldistearyl ammonium hectorite | manufactured by Elementis Specialties, Inc., BENTONE GEL PTM V solid content | 1.25 | 1.25 | 1.00 | 1.50 | 1.25 | 1.25 | 1.25 | 1.25 |
| | (B) | Methylphenylpolysiloxane | | 8.20 | 13.20 | 6.56 | 9.84 | 10.20 | 8.20 | 8.20 | 8.20 |
| | | Caprylyl methicone | manufactured by Momentive Performance Materials Japan LLC, SILSOFT 034 | 5.00 | | 5.00 | 5.00 | 5.00 | 5.00 | 5.00 | 5.00 |
| | (C) | Both-terminally alkyl (C16-18)-modified dimethylsiloxane-methyl(undecyl glyceryl ether)siloxane copolymer | Production Example 1 | 1.00 | 1.00 | 1.00 | 1.00 | 1.00 | 0.50 | 2.00 | - |
| | | Methylpolysiloxane-cetylmethylpolysiloxane-poly(oxyethylene-oxypropylene)methylpolysiloxane copolymer | manufactured by Evonik Industries AG, ABIL EM 90 | 2.50 | 2.50 | 2.50 | 2.50 | 2.50 | 2.00 | 3.00 | - |
| | Other components | PEG-10 dimethicone | | | | | | | | | 4.00 |
| | (D) | Diisostearyl malate | manufactured bv Nisshin OilliO Group, Ltd., COSMOL 222 | 7.00 | 7.00 | 7.00 | 7.00 | 7.00 | 7.00 | 7.00 | 7.00 |
| | | Neopentyl glycol dicaprate | manufactured bv Nisshin OilliO Group, Ltd., ESTEMOL N-01 | 5.50 | 5.50 | 7.50 | 3.50 | 3.50 | 6.50 | 4.00 | 5.00 |
| | | Triethyl citrate | | 0.55 | 0.55 | 0.44 | 0.66 | 0.55 | 0.55 | 0.55 | 0.55 |
| | Other components | Methylpolysiloxane | manufactured by Shin-Etsu Chemical Co., Ltd., Silicone KF-96L-2CS | 14.00 | 14.00 | 14.00 | 14.00 | 14.00 | 14.00 | 14.00 | 14.00 |

(continued)

| | | Component name | Starting material name | Example 1 | Example 2 | Example 3 | Example 4 | Example 5 | Example 6 | Example 7 | Comparative Example 1 |
|---|---|---|---|---|---|---|---|---|---|---|---|
| | | (Acrylates/Ethylhexyl acrylate/dimethicone methacrylate) copolymer | manufactured by Shin-Etsu Chemical Co., Ltd., KP-578 | 0.50 | 0.50 | 0.50 | 0.50 | 0.50 | 0.50 | 0.50 | 0.50 |
| Water phase | Other components | Dipropylene glycol | | 4.00 | 4.00 | 4.00 | 4.00 | 4.00 | 4.00 | 4.00 | 4.00 |
| | | 1,3-Butylene alvcol | | 6.00 | 6.00 | 6.00 | 6.00 | 6.00 | 6.00 | 6.00 | 6.00 |
| | | Dialycerin | | 1.00 | 1.00 | 1.00 | 1.00 | 1.00 | 1.00 | 1.00 | 1.00 |
| | | Sodium chloride | | 0.50 | 0.50 | 0.50 | 0.50 | 0.50 | 0.50 | 0.50 | 0.50 |
| | | Methyl p-hydroxybenzoate | | 0.20 | 0.20 | 0.20 | 0.20 | 0.20 | 0.20 | 0.20 | 0.20 |
| | | Phenoxyethanol | | 0.50 | 0.50 | 0.50 | 0.50 | 0.50 | 0.50 | 0.50 | 0.50 |
| | | Water-soluble collagen solution (3) | manufactured by Ichimaru Pharcos Co., Ltd. , NEPTIGEN N | 0.10 | 0.10 | 0.10 | 0.10 | 0.10 | 0.10 | 0.10 | 0.10 |
| | | Sodium hyaluronate (2) | manufactured by Kikkoman Biochemifa Company, Hyaluronic acid FCH(FCH-SU) | 0.01 | 0.01 | 0.01 | 0.01 | 0.01 | 0.01 | 0.01 | 0.01 |
| | | BHT | | 0.02 | 0.02 | 0.02 | 0.02 | 0.02 | 0.02 | 0.02 | 0.02 |
| | | Purified water | | 18.927 | 18.927 | 18.927 | 18.927 | 18.927 | 18.927 | 18.927 | 18.927 |
| Powder phase | Color pigment | n-Octylsilylated titanium oxide | manufactured by Daito Kasei Kogyo Co., Ltd., OTS-2 MP-40 | 5.00 | 5.00 | 5.00 | 5.00 | 5.00 | 5.00 | 5.00 | 5.00 |
| | | n-Octylsilylated Blue No. 1 aluminum lake | manufactured by Daito Kasei Koavo Co., Ltd., OTS-5 BC BLUE NO.1 AL LAKE | 0.003 | 0.003 | 0.003 | 0.003 | 0.003 | 0.003 | 0.003 | 0.003 |
| | | Isopropyl triisostearoyl titanate-modified Red No. 202 | manufactured by Daito Kasei Koavo Co., Ltd., ITT-10 BC Red No. 202 K | 0.04 | 0.04 | 0.04 | 0.04 | 0.04 | 0.04 | 0.04 | 0.04 |

(continued)

| | | Component name | Starting material name | Example 1 | Example 2 | Example 3 | Example 4 | Example 5 | Example 6 | Example 7 | Comparative Example 1 |
|---|---|---|---|---|---|---|---|---|---|---|---|
| | | Dimethicone-disodium stearoyl glutamate composite-treated titanium oxide | manufactured by Miyoshi Kasei, Inc., SA/NAI-TR-10 MIBRID COLOR POWDER | 8.00 | 8.00 | 8.00 | 8.00 | 8.00 | 8.00 | 8.00 | 8.00 |
| | | Dimethicone-disodium stearoyl glutamate composite-treated red iron oxide | manufactured by Miyoshi Kasei, Inc SA/NAI-R-10 MIBRID COLOR POWDER | 0.34 | 0.34 | 0.34 | 0.34 | 0.34 | 0.34 | 0.34 | 0.34 |
| | | Dimethicone-disodium stearoyl glutamate composite-treated black iron oxide | manufactured by Miyoshi Kasei, Inc.,, SA/NAI-B-10 MIBRID COLOR POWDER | 0.06 | 0.06 | 0.06 | 0.06 | 0.06 | 0.06 | 0.06 | 0.06 |
| | | Dimethicone-disodium stearoyl glutamate composite-treated yellow iron oxide | manufactured by Miyoshi Kasei, Inc., SA/NAI-Y-10 MIBRID COLOR POWDER | 1.00 | 1.00 | 1.00 | 1.00 | 1.00 | 1.00 | 1.00 | 1.00 |
| | | Chromium hydroxide | manufactured by Sensient Cosmetic Technoloaies SAS, UNIPURE GREEN LC 790 CF | 0.10 | 0.10 | 0.10 | 0.10 | 0.10 | 0.10 | 0.10 | 0.10 |
| | Other components | Amino acid-isopropyl triisostearoyl titanate composite-treated titanated mica | Amino acid-isopropyl triisostearoyl titanate composite-treated, manufactured by Merck KGaA, LUMINOUS GLOW | 3.70 | 3.70 | 3.70 | 3.70 | 3.70 | 3.70 | 3.70 | 3.70 |
| | | Silicic anhydride | manufactured by Momentive Performance Materials Japan LLC, HARMONIE LUXE 4 | 5.00 | 5.00 | 5.00 | 5.00 | 5.00 | 5.00 | 5.00 | 5.00 |

(continued)

| | | Component name | Starting material name | Example 1 | Example 2 | Example 3 | Example 4 | Example 5 | Example 6 | Example 7 | Comparative Example 1 |
|---|---|---|---|---|---|---|---|---|---|---|---|
| | | | POWDER (specific surface area: 1.3 $m^2/g$) | | | | | | | | |
| Total | | | | 100.00 | 100.00 | 100.00 | 100.00 | 100.00 | 100.00 | 100.00 | 100.00 |
| Total (A) | | | | 1.25 | 1.25 | 1.00 | 1.50 | 1.25 | 1.25 | 1.25 | 1.25 |
| Total (B) | | | | 13.20 | 13.20 | 11.56 | 14.84 | 15.20 | 13.20 | 13.20 | 13.20 |
| Total (C) | | | | 3.50 | 3.50 | 3.50 | 3.50 | 3.50 | 2.50 | 5.00 | - |
| Total (D) | | | | 13.05 | 13.05 | 14.94 | 11.16 | 11.05 | 14.05 | 11.55 | 12.55 |
| (A)/(C) | | | | 0.36 | 0.36 | 0.29 | 0.43 | 0.36 | 0.50 | 0.25 | - |
| (B)/(C) | | | | 3.77 | 3.77 | 3.30 | 4.24 | 4.34 | 5.28 | 2.64 | - |
| (B)/(D) | | | | 1.01 | 1.01 | 0.77 | 1.33 | 1.38 | 0.94 | 1.14 | 1.05 |
| Evaluation | | Absence of makeupwear-off after 6 hours | | 15 | 14 | 14 | 15 | 13 | 13 | 14 | 9 |
| | | Fit feeling to skin | | 15 | 15 | 15 | 14 | 13 | 13 | 15 | 8 |
| | | Emulsification stability (40°C, 1 month) | | A | A | B | A | A | A | B | C |

[Table 2]

| | | Component name | Starting material name | Example 8 |
|---|---|---|---|---|
| Oil phase | (A) | Dimethyldistearyl ammonium hectorite | manufactured by Elementis Specialties, Inc., BENTONE GEL PTM V solid content | 1.25 |
| | (B) | Methylphenylpolysiloxane | | 8.20 |
| | | Caprylyl methicone | manufactured by Momentive Performance Materials Japan LLC, SILSOFT 034 | 5.00 |
| | (C) | Both-terminally alkyl (C16-18)-modified dimethylsiloxane-methyl(undecyl glyceryl ether)siloxane copolymer | Production Example 1 | 0.50 |
| | | Methylpolysiloxane-cetylmethylpolysiloxane-poly(oxyethylene-oxypropylene)methylpolysiloxane copolymer | manufactured by Evonik Industries AG, ABIL EM 90 | 2.50 |
| | | dimethylsiloxane-methyl(undecyl glyceryl ether)siloxane copolymer | | 1.00 |
| | (D) | Diisostearyl malate | manufactured by Nisshin OilliO Group, Ltd., COSMOL 222 | 6.73 |
| | | Neopentyl glycol dicaprate | manufactured by Nisshin OilliO Group, Ltd., ESTEMOL N-01 | 5.00 |
| | | Triethyl citrate | | 0.55 |
| | Other components | Methylpolysiloxane | manufactured by Shin-Etsu Chemical Co., Ltd., Silicone KF-96L-2CS | 14.00 |
| | | (Acrylates/Ethylhexyl acrylate/-dimethicone methacrylate) copolymer | manufactured by Shin-Etsu Chemical Co., Ltd., KP-578 | 0.50 |
| Water phase | Other components | Dipropylene alycol | | 4.00 |
| | | 1,3-Butylene glycol | | 6.00 |
| | | Dialycerin | | 1.00 |
| | | Sodium chloride | | 0.50 |
| | | Methyl p-hydroxybenzoate | | 0.20 |
| | | Phenoxyethanol | | 0.50 |
| | | Water-soluble collagen solution (3) | manufactured by Ichimaru Pharcos Co., Ltd. , NEPTIGEN N | 0.10 |
| | | Sodium hyaluronate (2) | manufactured by Kikkoman Biochemifa Company, Hyaluronic acid FCH(FCH-SU) | 0.01 |
| | | BHT | | 0.025 |
| | | Purified water | | 18.992 |

(continued)

| | | Component name | Starting material name | Example 8 |
|---|---|---|---|---|
| Powder phase | Color pigment | n-Octylsilylated titanium oxide | manufactured by Daito Kasei Koavo Co., Ltd., OTS-2 MP-40 | 5.362 |
| | | n-Octylsilylated Blue No. 1 aluminum lake | manufactured by Daito Kasei Koavo Co., Ltd., OTS-5 BC BLUE NO.1 AL LAKE | 0.003 |
| | | Isopropyl triisostearoyl titanate-modified Red No. 202 | manufactured by Daito Kasei Kogyo Co., Ltd., ITT-10 BC Red No. 202 K | 0.048 |
| | | Dimethicone-disodium stearoyl glutamate composite-treated titanium oxide | manufactured by Miyoshi Kasei, Inc., SA/NAI-TR-10 MIBRID COLOR POWDER | 8.00 |
| | | Dimethicone-disodium stearoyl glutamate composite-treated red iron oxide | manufactured by Miyoshi Kasei, Inc., SA/NAI-R-10 MIBRID COLOR POWDER | 0.34 |
| | | Dimethicone-disodium stearoyl glutamate composite-treated black iron oxide | manufactured by Miyoshi Kasei, Inc., SA/NAI-B-10 MIBRID COLOR POWDER | 0.06 |
| | | Dimethicone-disodium stearoyl glutamate composite-treated yellow iron oxide | manufactured by Miyoshi Kasei, Inc., SA/NAI-Y-10 MIBRID COLOR POWDER | 0.92 |
| | | Chromium hydroxide | manufactured by Sensient Cosmetic Technologies SAS, UNIPURE GREEN LC 790 CF | 0.10 |
| | Other components | Amino acid-isopropyl triisostearoyl titanate composite-treated titanated mica | Amino acid-isopropyl triisostearoyl titanate composite-treated, manufactured by Merck KGaA, LUMINOUS GLOW | 3.61 |
| | | Silicic anhydride | manufactured by Momentive Performance Materials Japan LLC, HARMONIE LUXE 4 POWDER (specific surface area: 1.3 $m^2/g$) | 5.00 |
| Total | | | | 100.00 |
| Total (A) | | | | 1.25 |
| Total (B) | | | | 13.20 |
| Total (C) | | | | 400 |
| Total (D) | | | | 12.28 |
| (A)/(C) | | | | 0.31 |
| (B)/(C) | | | | 3.30 |
| (B)/(D) | | | | 1.07 |
| Evaluation | | Absence of makeup wear-off after 6 hours | | 15 |
| | | Fit feeling to skin | | 15 |
| | | Emulsification stability (40°C, 1 month) | | A |

The water-in-oil emulsion cosmetics (liquid foundations) of Examples 1 to 8 can be used by filling each cosmetic into a container such as a bottle container or a tube container, without impregnation of a holder, and taking the cosmetic by the finger or an applicator directly from the container to apply the cosmetic to the skin. In this case as well, makeup durability

and fit feeling to the skin are excellent, and emulsification stability is also excellent.

**Claims**

1. A water-in-oil emulsion composition for impregnation comprising the following components (A), (B), (C) and (D):

   (A) cation-modified clay mineral substituted with a quaternary ammonium ion,
   (B) one or more members selected from the group consisting of phenyl-modified silicone and alkyl-modified silicone which are in a liquid state at 25°C,
   (C) modified silicone containing an alkyl group having 10 or more carbon atoms and a polyglyceryl group, and
   (D) an ester oil which is in a liquid state at 25°C.

2. A cosmetic in which a holder impregnated with a water-in-oil emulsion composition according to claim 1 is housed in a compact container.

3. The cosmetic according to claim 2, wherein the component (C) comprises both-terminally alkyl-modified glyceryl ether-modified silicone.

4. The cosmetic according to claim 2, wherein the component (C) comprises alkyl glyceryl ether-modified silicone of the formula (2):

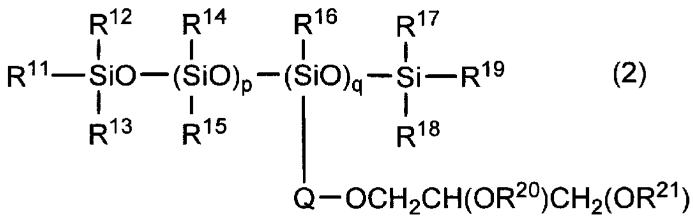

(2)

   wherein $R^{11}$ and $R^{19}$ are the same or different and are each independently a linear or branched hydrocarbon group having 1 to 32 carbon atoms in terms of a mode value; $R^{12}$ to $R^{18}$ are the same or different and are each independently a linear or branched hydrocarbon group having 1 to 5 carbon atoms; Q is a linear or branched divalent hydrocarbon group having 3 to 20 carbon atoms; $R^{20}$ and $R^{21}$ are each independently a hydrogen atom or a linear or branched hydrocarbon group having 1 to 28 carbon atoms, and at least one of them is a hydrogen atom; p represents the number of repeat units and is a number of 5 or more and 60 or less as an average value; q represents the number of repeat units and is a number of 2.5 or more and 10 or less as an average value; and constituent units with the number of repeats, p and q, are any of block copolymers and random copolymers, provided that the formula has one or more alkyl groups having 10 or more carbon atoms.

5. The cosmetic according to any one of claims 2 to 4, wherein a content of the component (A) is from 0.1 to 8% by mass, a content of the component (B) is from 1 to 30% by mass, a content of the component (C) is from 0.1 to 12% by mass, and a content of the component (D) is from 3 to 40% by mass.

6. The cosmetic according to any one of claims 2 to 5, wherein a mass ratio of the component (A) to the component (C), (A)/(C), is from 0.05 to 3.

7. The cosmetic according to any one of claims 2 to 6, wherein a mass ratio of the component (B) to the component (C), (B)/(C), is from 0.5 to 15.

8. The cosmetic according to any one of claims 2 to 7, wherein a viscosity at 25°C of the component (D) is 10 000 mPa·s or less.

9. The cosmetic according to any one of claims 2 to 8, wherein a mass ratio of the component (B) to the component (D), (B)/(D), is from 0.2 to 5.

10. The cosmetic according to any one of claims 2 to 9, wherein the water-in-oil emulsion composition further comprises from 1 to 30% by mass of a hydrophobized color pigment.

**11.** The cosmetic according to any one of claims 2 to 10, wherein the water-in-oil emulsion composition further comprises from 1 to 50% by mass of a volatile oil.

**12.** The cosmetic according to any one of claims 2 to 11, wherein a content of water in the water-in-oil emulsion composition is from 3 to 50% by mass.

**13.** The cosmetic according to any one of claims 2 to 12, wherein a viscosity at 30°C of the water-in-oil emulsion composition is from 3 000 to 30 000 mPa·s.

**14.** The cosmetic according to any one of claims 2 to 13, wherein the holder is a foam, a porous material, or an assembly of fibers.

**15.** The cosmetic according to any one of claims 2 to 14, wherein the holder is an assembly of polyester fibers.

**16.** A method for using a cosmetic according to any one of claims 2 to 15, comprising taking a water-in-oil emulsion composition impregnated into a holder by finger or an applicator from the holder, and applying it to the skin

**17.** Use of a water-in-oil emulsion composition comprising the following components (A), (B), (C) and (D) :

(A) cation-modified clay mineral substituted with a quaternary ammonium ion,
(B) one or more members selected from the group consisting of phenyl-modified silicone and alkyl-modified silicone which are in a liquid state at 25°C,
(C) modified silicone containing an alkyl group having 10 or more carbon atoms and a polyglyceryl group, and
(D) an ester oil which is in a liquid state at 25°C as a makeup cosmetic comprising a holder impregnated with the water-in-oil emulsion composition.

**18.** A cosmetic kit comprising a set of a holder impregnated with a water-in-oil emulsion composition, and a compact container, the water-in-oil emulsion composition comprising the following components (A), (B), (C) and (D):

(A) cation-modified clay mineral substituted with a quaternary ammonium ion,
(B) one or more members selected from the group consisting of phenyl-modified silicone and alkyl-modified silicone which are in a liquid state at 25°C,
(C) modified silicone containing an alkyl group having 10 or more carbon atoms and a polyglyceryl group, and
(D) an ester oil which is in a liquid state at 25°C.

**19.** A water-in-oil emulsion composition comprising the following components (A), (B), (C) and (D) :

(A) cation-modified clay mineral substituted with a quaternary ammonium ion,
(B) one or more members selected from the group consisting of phenyl-modified silicone and alkyl-modified silicone which are in a liquid state at 25°C,
(C) modified silicone containing an alkyl group having 10 or more carbon atoms and a polyglyceryl group, and
(D) an ester oil which is in a liquid state at 25°C.

**INTERNATIONAL SEARCH REPORT**

International application No. **PCT/JP2024/028456**

**A. CLASSIFICATION OF SUBJECT MATTER**

***A61K 8/19***(2006.01)i; ***A61K 8/06***(2006.01)i; ***A61K 8/37***(2006.01)i; ***A61K 8/891***(2006.01)i; ***A61K 8/892***(2006.01)i; ***A61K 8/893***(2006.01)i; ***A61Q 1/02***(2006.01)i
FI: A61K8/19; A61K8/06; A61K8/37; A61K8/891; A61K8/892; A61K8/893; A61Q1/02

According to International Patent Classification (IPC) or to both national classification and IPC

**B. FIELDS SEARCHED**

Minimum documentation searched (classification system followed by classification symbols)

A61K8/19; A61K8/06; A61K8/37; A61K8/891; A61K8/892; A61K8/893; A61Q1/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y<br><br>A | WO 2022/102579 A1 (SHIN-ETSU CHEMICAL CO., LTD.) 19 May 2022 (2022-05-19)<br>claims 1-8, example 11 | 1-3, 5-18<br><br>4 |
| Y<br><br>A | WO 2020/157956 A1 (SHISEIDO COMPANY, LTD.) 06 August 2020 (2020-08-06)<br>claims 1-4, paragraphs [0002], [0047], [0080], examples 1-8 | 1-3, 5-18<br><br>4 |
| A | JP 2022-159775 A (ARUBION KK) 18 October 2022 (2022-10-18)<br>claims 1-12, paragraphs [0035], [0048], example 28 | 1-18 |
| A | JP 2022-190400 A (KAO CORPORATION) 26 December 2022 (2022-12-26)<br>entire text, all drawings | 1-18 |
| A | JP 2023-098478 A (KOSE CORPORATION) 10 July 2023 (2023-07-10)<br>entire text, all drawings | 1-18 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 October 2024** | **05 November 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (July 2022)

INTERNATIONAL SEARCH REPORT

International application No.
**PCT/JP2024/028456**

**C. DOCUMENTS CONSIDERED TO BE RELEVANT**

| Category* | Citation of document, with indication, where appropriate, of the relevant passages | Relevant to claim No. |
|---|---|---|
| A | JP 2022-030780 A (POLA CHEMICAL INDUSTRIES, INC.) 18 February 2022 (2022-02-18) entire text, all drawings | 1-18 |
| A | JP 2022-163970 A (SHISEIDO CO., LTD.) 27 October 2022 (2022-10-27) entire text, all drawings | 1-18 |

Form PCT/ISA/210 (second sheet) (July 2022)

**INTERNATIONAL SEARCH REPORT**

International application No.
**PCT/JP2024/028456**

**Box No. III Observations where unity of invention is lacking (Continuation of item 3 of first sheet)**

This International Searching Authority found multiple inventions in this international application, as follows:

Document 1: WO 2022/102579 A1 (SHIN-ETSU CHEMICAL CO., LTD.) 19 May 2022 (2022-05-19)
claims 1-8, example 11, comparative example 7

The claims are classified into the following two inventions.

(Invention 1) Claims 1-18

Document 1 sets forth a water-in-oil-type emulsion composition containing disteardimonium hectorite (dimethyl distearyl ammonium hectorite), which is an organically modified clay mineral (corresponding to component (A) in the present application), a crosslinked alkyl-modified silicone mixture such as KSG-42A, KSG-310, KSG-41A, or KF-6048 (corresponding to component (B) in the present application), a modified silicone having alkyl groups of 10 or more carbons and polyglycerol groups, such as KF-6105 or KSG-830 (corresponding to component (C) in the present application), and ethylhexyl palmitate or isotridecyl isononanoate (corresponding to component (D) in the present application) (claims 1-8, example 11, comparative example 7). This being the case, the invention in claims 1-16 of the present application has the special technical feature of "being for impregnation," and thus is classified as invention 1.

The inventions in claims 17 and 18 respectively specify that "the water-in-oil-type emulsion composition is impregnated in a holder" and the invention has "a holder in which the water-in-oil-type emulsion composition has been impregnated." Since said inventions are substantially identical to or similarly closely related to claim 1, which is "a water-in-oil-type emulsion composition for impregnation," said claims are classified as invention 1.

(Invention 2) Claim 19

Claim 19 does not specify that the water-in-oil-type emulsion composition is "for impregnation," and cannot be said to share a same or corresponding special technical feature with claim 1 classified as invention 1. Accordingly, since claim 19 cannot be classified as invention 1, said claim is classified as invention 2.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Claims 1-18**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.
**PCT/JP2024/028456**

| Patent document cited in search report | Publication date (day/month/year) | Patent family member(s) | Publication date (day/month/year) |
|---|---|---|---|
| WO 2022/102579 A1 | 19 May 2022 | US 2024/0016724 A1<br>claims 1-8, example 11<br>EP 4245377 A1<br>CN 116472026 A | |
| WO 2020/157956 A1 | 06 August 2020 | CN 111801092 A | |
| JP 2022-159775 A | 18 October 2022 | (Family: none) | |
| JP 2022-190400 A | 26 December 2022 | (Family: none) | |
| JP 2023-098478 A | 10 July 2023 | (Family: none) | |
| JP 2022-030780 A | 18 February 2022 | (Family: none) | |
| JP 2022-163970 A | 27 October 2022 | WO 2022/220100 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013530252 A
- JP 2017088631 A
- JP 2019131489 A
- JP 4134013 A
- JP 4195043 B